# EUROPEAN PATENT SPECIFICATION

(11) **EP 4 186 479 B1**
(45) Date of publication and mention of the grant of the patent: **02.07.2025**
(21) Application number: 22208758.7
(22) Date of filing: 22.11.2022
(51) Int. Cl.: A61F 7/02, A61F 7/00

(54) **HEADWEAR FOR USE IN THE ALLEVIATION OF TENSION-TYPE HEADACHES, MIGRAINE, AND OTHER TYPES OF CEPHALALGIA**
KOPFBEDECKUNG ZUR LINDERUNG VON SPANNUNGSKOPFSCHMERZEN, MIGRÄNE UND ANDEREN CEPHALALGIEARTEN
COUVRE-CHEF DESTINÉ À ÊTRE UTILISÉ DANS L'ATTÉNUATION DE MAUX DE TÊTE DE TYPE À TENSION, DE MIGRAINES ET D'AUTRES TYPES DE CÉPHALAGE

(30) Priority: 24.11.2021 DK PA202101113
(43) Date of publication of application: 31.05.2023
(73) Proprietor: Noyocare ApS, 9500 Hobro (DK)
(72) Inventor: Andersen, Kristian, 9500 Hobro (DK); Stilling, Katarina, 9500 Hobro (DK)
(74) Representative: Larsen & Birkeholm A/S

(56) References cited:
- FR-A1- 2 852 231
- US-A- 4 765 338
- US-A- 5 314 456
- US-A- 5 480 418
- US-A1- 2008 228 248
- US-A1- 2015 141 903

## Description

### Technical field of the invention

The present invention relates to headwear for use in the alleviation of tension-type headaches, migraine, and other types of cephalalgia.

### Background of the invention

The public health significance of headache pain (cephalalgia) and occipital neuralgia is often overlooked, most likely because of their episodic nature and the lack of mortality attributed to them. Headache disorders and occipital neuralgia are, however, often incapacitating, with considerable impact on social activities and work, and may lead to significant consumption of drugs with adverse side effects.

The International Headache Society (IHS) has identified 13 different general groupings of headache, such as tension-type headache and migraine. Migraine symptoms may overlap considerably with that of tension-type headaches. Tension-type headaches are believed by some experts to be a mild variant of migraine headache. Patients with tension-type headaches who also have migraines may experience nausea and vomiting with a tension headache, though when they do, it typically is mild and for a shorter duration compared to that with a migraine. Tension-type headache may however be a disorder in individuals who do not have migraine and may manifest as attacks of mild migraine in individuals with migraines. Migraine is a debilitating condition that is usually characterized by a throbbing pain on one or both sides of the head often accompanied by other neurological symptoms, such as visual disturbances, nausea, vomiting, dizziness, extreme sensitivity to sound, light, touch, and smell, and tingling or numbness in the extremities or face. Migraine attacks vary in severity from person to person and are believed to affect over 10 percent of the population worldwide.

Migraine headaches are typically the result of specific cerebral vasodilatations that press on and activate certain afferent pain receptors and pain receptor neural networks. In most cases, the migraine patients are medicated. Some patients may try using heat treatment on some of the muscles on the head, but it is difficult to direct the heat at the right places, why very few patients use such methods.

Thus, it is an object of the present invention to provide a device that can alleviate tension-type headaches, migraine, and other types of cephalalgia. US2008228248 and US5314456 are prior art devices, being used to treat migraines.

### Description of the invention

The design of the present invention facilitates an efficient, precise, and comfortable heat-treatment of specific muscles and nerves in the facial region and the neck region. The invention is disclosed in independent claim 1. Preferred embodiments are disclosed in the dependent claims.

One aspect relates to a headwear for use in the alleviation of tension-type headaches, migraine, and other types of headaches, said headwear comprising:
- a textile selected from cotton, wool, linen, hemp, and mixtures thereof;
- a system of compartments formed in or on said textile, each compartment containing a material suitable for being heated in a microwave oven to a temperature of within the range of 40-60 degrees Celsius; and
- a fastening means adapted for approximating the textile to the curvature of a user's head;
wherein said system of compartments each are configured to target specific muscles and/or nerves on the user's head with heat within the range of 40-60 degrees Celsius, wherein said system of compartments comprises:
- a pair of compartments of a first type positioned on said headwear to target the masseter muscles and/or the trigeminus nerve with heat within the range of 40-60 degrees Celsius, preferably within the range of 50-55 degrees Celsius;
- a pair of compartments of a second type positioned on said headwear to target the occipitalis muscles and/or the occipital nerve with heat within the range of 40-60 degrees Celsius, preferably within the range of 50-55 degrees Celsius.

The International Headache Society (IHS) classifies cephalalgia into two main categories: primary and secondary. The main difference between these two categories is that the secondary type headaches/cephalalgia are attributed to a particular cause.

Primary Headaches includes migraine, tension-type headache, cluster headache, and other primary headaches. Secondary Headaches includes headache attributed to head and/or neck trauma, headache attributed to cranial or cervical vascular disorder, headache attributed to non-vascular intracranial disorder, headache attributed to a substance or its withdrawal, headache attributed to infection, headache attributed to disorder of homeostasis, headache or facial pain attributed to disorder of cranium, neck, eyes, ears, nose, sinuses, teeth, mouth or other facial or cranial structures, and headache attributed to psychiatric disorder.

The inventor of the present invention has found that heat stimulation of especially the masseter muscles and/or the trigeminus nerve and the occipitalis muscles and/or the occipital nerve results in the alleviation of tension-type headaches, migraine, and other types of headaches/cephalalgia. Although the overall mechanisms and specific pathways responsible for primary and secondary headaches are still being elucidated, it is well known that many patients that experience these types of headaches often feel pain in both the front and the back of the head. While the front of the head is innervated, among others, by the ophthalmic branch of the trigeminal nerve, the back of the head is mainly innervated by spinal branches arising from C1 through C4, which are the first four cervical spinal nerves and which, among others, form the occipital and suboccipital nerves. However, recent studies suggest that there is a functional connection between the branches of the nervous system that innervate the front and the back of the head. Without being bound by any specific theory, it is believed that many types of headaches/cephalalgia are associated with nociceptive pathways of the trigeminal nerve, and that occipital afferents and trigeminal afferents converge in the lower brain stem/upper cervical region, such as the trigeminal cervical complex. Via this convergence, it is believed that heat stimulation of the occipitalis muscles and/or the occipital nerve may have an inhibitory effect on nociception transmitted via trigeminal afferents, which may then have a pain-relieving effect on migraine or other types of headaches.

In one or more embodiments, the system of compartments further comprises:
- a pair of compartments of a third type positioned on said headwear to target the temporal muscle with heat within the range of 40-60 degrees Celsius, preferably within the range of 50-55 degrees Celsius.

In one or more embodiments, the system of compartments further comprises:
- a compartment of a fourth type positioned on said headwear to target the splenius capitis muscles (and possibly also parts of the occipital nerve passing that region) with heat within the range of 40-60 degrees Celsius, preferably within the range of 50-55 degrees Celsius. Splenius Capitis Muscle Syndrome is a commonly occurring headache, neck ache, and facial pain disorder that typically mimics the respective pain patterns of temporal tendonitis and migraine headache. The reference areas of pain include rear of the head aches and hurts, lateral temple headache, retro-orbital headache and pressure, aching pain above the eye, aching pain at cheekbone under eye, eye hurts and is sensitive to bright light, pain radiating to neck, shoulder and arm at times, and nausea and vomiting when pain is intense. The onset of pain is often caused by motor vehicular trauma, blunt trauma, falls, and, in particular, postural situations where superior and inferior lateral oblique movements of the head on the neck occur.

In one or more embodiments, the system of compartments further comprises:
- one or more compartments of a fourth type positioned on said headwear to target the splenius capitis muscles, the sternocleidomastoid muscles, the superior part of the trapezius muscle and/or the occipital nerve with heat within the range of 40-60 degrees Celsius. Trapezius muscle spasticity may cause tension headaches. The spinal accessory and occipital nerves pass through this muscle. Spasms or increased tone of trapezius can put pressure on these nerves causing a headache. The headache has a typical "ram's horn" distribution causing throbbing pain on both sides of the head, traveling from the region at the back of the head and wrapping around to the top of the head and forehead region. Pain in the sternocleidomastoid muscle can cause neck tenderness and headaches. A person with sternocleidomastoid muscle pain might notice trigger points along the side or front of the neck. Frequently, however, pain from this muscle radiates elsewhere, causing ear, eye, or sinus pain.

The material suitable for being heated in a microwave oven to a temperature of within the range of 40-60 degrees Celsius may be many different types of materials, such as fruit stones (e.g., cherry stones (pits), walnut shells, olive stones, and peach pit), jasmine rice, whey kernels, flax seeds, and the like. Preferably, the material is selected from cherry stones, walnut shells, peach pit, and mixtures thereof, as these materials allow for washing of the headwear with water.

In one or more embodiments, the headwear further comprises two zones defining the areas corresponding to the user's ears, said zones made solely of said textile, thereby preventing heating of said ears, and also allows the user to hear when wearing the headwear.

In one or more embodiments, the headwear is configured with an opening defining the areas corresponding to at least a part of the user's frontal, mid, and crown scalp regions. This configuration prevents the user from overheating.

The compartments formed in the textile may be configured as a pocket, e.g., a pocket that can be closed and opened, e.g., with a plastic zipper or hook and loop fastening means, thereby allowing the user to exchange the material suitable for being heated in a microwave oven to a temperature of within the range of 40-60 degrees Celsius. The material may be included in a pouch adapted for fitting within the pocket. Alternatively, the compartments are closed, i.e., not able to be opened under normal use conditions. In this situation, the material suitable for being heated in a microwave oven to a temperature of within the range of 40-60 degrees Celsius is introduced during the production of the headwear.

Fold lines may be formed or sewn in the textile that are adapted for approximating the textile to the curvature of the user's head.

As used in the specification and the appended claims, the singular forms "a", "an", and "the" include plural referents unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about", it will be understood that the particular value forms another embodiment.

It should be noted that embodiments and features described in the context of one of the aspects of the present invention also apply to the other aspects of the invention.

### Brief description of the figures

Figures 1A-C show a headwear in accordance with various embodiments of the invention placed on the head of a user.
Figure 2 shows an unfolded headwear in accordance with various embodiments of the invention.

### Detailed description of the invention

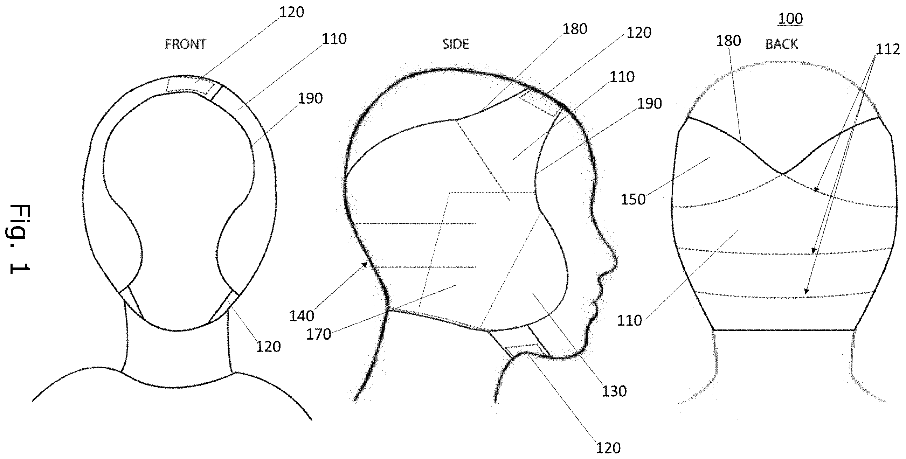

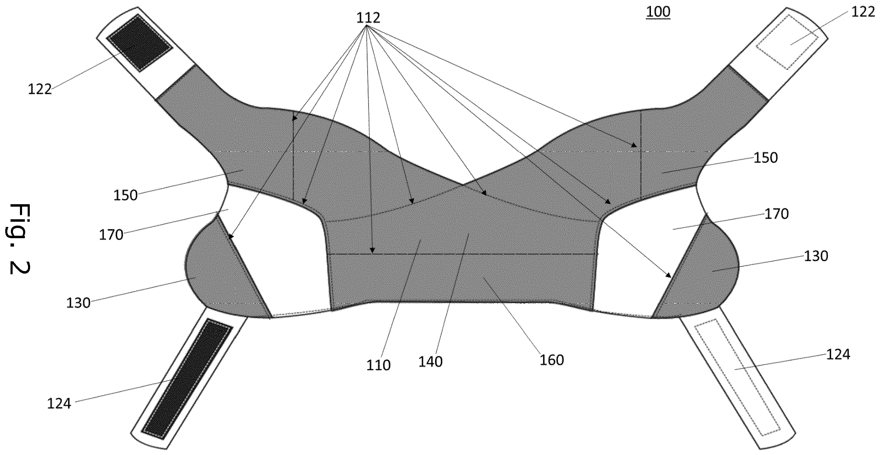

The following description is to be seen as non-limiting examples of a headwear 100 according to various embodiments of the present invention. Referring to Figure 1, the general scheme of the invention is shown, where a user is wearing the headwear 100 and may be seen from the front, side, and back. Figure 2 shows the headwear 100 unfolded. The headwear 100 comprises a textile 110 selected from cotton, wool, linen, hemp, and mixtures thereof. A system of compartments is formed in or on the textile 110. Each compartment contains a material suitable for being heated in a microwave oven to a temperature of within the range of 40-60 degrees Celsius. Suitable materials could be cherry stones or walnut shells, both materials being able to withstand the heating without noticeable degradation. Furthermore, the materials are capable of holding the temperature for about 10-15 minutes, thereby securing a proper treatment period without the risk of damaging the tissue due to excessive heat treatment time. The system of compartments is configured to target specific muscles and/or nerves on the user's head with heat within the range of 40-60 degrees Celsius. The system of compartments is shown with four different compartments. A pair of compartments of a first type 130 positioned on the headwear 100 to target the masseter muscles and/or the trigeminus nerve with heat; a pair of compartments of a second type 140 positioned on the headwear 100 to target the occipitalis muscles and/or the occipital nerve with heat; a pair of compartments of a third type 150 positioned on the headwear 100 to target the temporal muscle with heat; and a compartment of a fourth type 160 positioned on the headwear 100 to target the splenius capitis muscles, the sternocleidomastoid muscles, the superior part of the trapezius muscle, and the occipital nerve with heat. Hence, the extent and position of the compartments are very precise to avoid heating unwanted regions. One such region is the ears, why two zones 170 defining the areas corresponding to the user's ears are made solely of said textile. Furthermore, the headwear 100 is configured with a first opening 180 defining the areas corresponding to a part of the user's frontal, mid, and crown scalp regions; and a second opening 190 defining the areas corresponding to at least a part of the user's forehead, orbital, nasal, and oral regions. The openings are also present to avoid heating unwanted head regions.

The headwear 100 comprises fastening means 120 adapted for approximating the textile 110 to the curvature of the user's head. In Figure 2, the fastening means are easier seen, and comprises a first 122 and a second 124 pair of fastening straps, both configured with hook and loop fasteners. The first pair of fastening straps 122 are flanking both the first 180 and the second 190 openings, while the second pair of fastening straps 124 are flanking only the second opening 190.

One way of using the headwear could be as follows. The first step using the headwear 100 is to fit it to the head. It must be folded around the head so that the first fastening straps 122 are fastened and fitted across the frontal scalp of the user, thereby creating the first opening 180 in the headwear. Then the second pair of fastening straps 124 are fastened and fitted below the chin of the user. The zones 170 are to be located over the ears, as there is no heat therein. After fitting the headwear 100, the second fastening strap 124 is opened again, while keeping the first fastening straps 122 closed, and the headwear is removed from the user's head. The headwear 100 is then placed in a microwave-oven for short time, such as about 30-60 seconds at 600 watts until the temperature of the material filled compartments is about 55 degrees Celsius. A temperature indicator may be attached to the compartments to secure the correct temperature. Alternatively, if it is too hot, it may be left to cool for some minutes. When the temperature is right, the headwear 100 is put on the head of the user and fastened thereto by fastening the second pair of fastening straps 124.

### References

- 100: Headwear
- 110: Textile
- 112: Fold line
- 120: Fastening means
- 122: First pair of fastening straps
- 124: Second pair of fastening straps
- 130: Compartment of a first type
- 140: Compartment of a second type
- 150: Compartment of a third type
- 160: Compartment of a fourth type
- 170: Zone
- 180: First opening
- 190: Second opening

## Claims

1. A headwear (100) for use in the alleviation of tension-type headaches, migraine and other types of cephalalgia, said headwear comprising:
- a textile (110);
- a system of compartments formed in or on said textile (110), each compartment containing a material suitable for being heated in a microwave oven to a temperature of within the range of 40-60 degrees Celsius; and
- a fastening means (120) adapted for approximating the textile to the curvature of a user's head;
wherein said system of compartments is configured to target specific muscles and/or nerves on the user's head with heat, wherein said system of compartments comprises a pair of compartments of a second type (140) positioned on said headwear (100) to target the occipitalis muscles and the occipital nerve with heat;
**characterized in that** said system of compartments is configured to target specific muscles and/or nerves on the user's head with heat within the range of 40-60 degrees Celsius; said system of compartments further comprising a pair of compartments of a first type (130) positioned on said headwear (100) to target the masseter muscles and the trigeminus nerve; and wherein the textile (110) is selected from cotton, wool, linen, hemp, and mixtures thereof.

2. The headwear (100) according to claim 1, wherein the system of compartments further comprises:
- a pair of compartments of a third type (150) positioned on said headwear (100) to target the temporal muscle with heat within the range of 40-60 degrees Celsius.

3. The headwear (100) according to any one of the claims 1-2, wherein the system of compartments further comprises:
- a compartment of a fourth type (160) positioned on said headwear to target the splenius capitis muscles with heat within the range of 40-60 degrees Celsius.

4. The headwear (100) according to any one of the claims 1-2, wherein the system of compartments further comprises:
- one or more compartments of a fourth type (160) positioned on said headwear (100) to target the splenius capitis muscles, the sternocleidomastoid muscles, the superior part of the trapezius muscle and/or the occipital nerve with heat within the range of 40-60 degrees Celsius.

5. The headwear (100) according to any one of the claims 1-4, further comprising two zones (170) defining the areas corresponding to the user's ears, said zoned made solely of said textile, thereby preventing heating of said ears.

6. The headwear (100) according to any one of the claims 1-5, configured with a first opening (180) defining the areas corresponding to at least a part of the user's frontal, mid, and crown scalp regions.

7. The headwear (100) according to claim 6, wherein the fastening means (120) adapted for approximating the textile (110) to the curvature of a user's head comprises a first pair of fastening straps (122), preferably configured with hook and loop fasteners, and wherein said first pair of fastening straps (122) are flanking said first opening (180).

8. The headwear (100) according to any one of the claims 1-6, configured with a second opening (190) defining the areas corresponding to at least a part of the user's forehead, orbital, nasal, and oral regions.

9. The headwear (100) according to claim 8, wherein the fastening means (120) adapted for approximating the textile to the curvature of a user's head comprises a second pair of fastening straps (124), preferably configured with hook and loop fasteners, and wherein said second pair of fastening straps (124) are flanking said second opening (190).

10. The headwear (100) according to any one of the claims 1-9, wherein the textile (110) comprises fold lines (170) adapted for approximating the textile (110) to the curvature of the user's head.

## Patentansprüche

1. Eine Kopfbedeckung (100) zur Verwendung bei der Linderung von Spannungskopfschmerzen, Migräne und anderen Arten von Cephalalgie, wobei die Kopfbedeckung umfasst:
eine Textilie (110);
ein System von Fächern, die in oder auf dem Textil (110) ausgebildet sind, wobei jedes Fach ein Material enthält, das geeignet ist, in einem Mikrowellenofen auf eine Temperatur im Bereich von 40-60 Grad Celsius erhitzt zu werden; und
ein Befestigungsmittel (120), das geeignet ist, das Textil an die Krümmung des Kopfes eines Benutzers anzupassen;
wobei das System von Fächern so konfiguriert ist, dass es spezifische Muskeln und/oder Nerven am Kopf des Benutzers mit Wärme behandelt, wobei das System von Fächern ein Paar von Fächern eines zweiten Typs (140) umfasst, die auf der Kopfbedeckung (100) positioniert sind, um die Okzipitalis-Muskeln und den Okzipitalnerv mit Wärme behandeln;
**dadurch gekennzeichnet, dass** das System von Fächern so konfiguriert ist, dass es auf spezifische Muskeln und/oder Nerven am Kopf des Benutzers mit Wärme im Bereich von 40-60 Grad Celsius abzielt; wobei das System von Fächern ferner ein Paar von Fächern eines ersten Typs (130) umfasst, die auf der Kopfbedeckung (100) positioniert sind, um auf die Massetermuskeln und den Trigeminusnerv abzuzielen; und wobei das Textil (110) aus Baumwolle, Wolle, Leinen, Hanf und Mischungen davon besteht.

2. Kopfbedeckung (100) nach Anspruch 1, wobei das System von Fächern weiterhin umfasst:
ein Paar von Fächern eines dritten Typs (150), die auf der Kopfbedeckung (100) angeordnet sind, um den Schläfenmuskel mit Wärme im Bereich von 40-60 Grad Celsius zu behandeln.

3. Kopfbedeckung (100) nach Anspruch 1, wobei das System von Fächern ferner umfasst:
ein viertes Fach (160), das an der Kopfbedeckung angeordnet ist, um die Muskeln des Splenius capitis mit Wärme im Bereich von 40 bis 60 Grad Celsius zu behandeln.

4. Kopfbedeckung (100) nach einem der Ansprüche 1 bis 2, wobei das System von Fächern ferner umfasst:
ein oder mehrere Fächer eines vierten Typs (160), die an der Kopfbedeckung (100) angeordnet sind, um die Muskeln Splenius capitis, Sternocleidomastoideus, den oberen Teil des Trapezmuskels und/oder den Okzipitalnerv mit Wärme im Bereich von 40 bis 60 Grad Celsius zu behandeln.

5. Kopfbedeckung (100) nach einem der Ansprüche 1 bis 4, die außerdem zwei Zonen (170) umfasst, die die Bereiche definieren, die den Ohren des Benutzers entsprechen, wobei die Zonen ausschließlich aus dem Textil bestehen, wodurch eine Erwärmung der Ohren verhindert wird.

6. Kopfbedeckung (100) nach einem der Ansprüche 1 bis 5, die mit einer ersten Öffnung (180) versehen ist, die die Bereiche definiert, die zumindest einem Teil der Kopfhaut des Benutzers im vorderen, mittleren und oberen Bereich entsprechen.

7. Kopfbedeckung (100) nach Anspruch 6, wobei die Befestigungsmittel (120), die zur Annäherung des Textils (110) an die Krümmung des Kopfes eines Benutzers geeignet sind, ein erstes Paar von Befestigungsbändern (122) umfassen, die vorzugsweise mit Klettverschlüssen konfiguriert sind, und wobei das erste Paar von Befestigungsbändern (122) die erste Öffnung (180) flankiert.

8. Kopfbedeckung (100) nach einem der Ansprüche 1 bis 6, die mit einer zweiten Öffnung (190) versehen ist, die die Bereiche definiert, die mindestens einem Teil der Stirn-, Augenhöhlen-, Nasen- und Mundregion des Benutzers entsprechen.

9. Kopfbedeckung (100) nach Anspruch 8, wobei die Befestigungsmittel (120), die dazu ausgelegt sind, das Textil an die Krümmung des Kopfes eines Benutzers anzupassen, ein zweites Paar Befestigungsbänder (124) umfassen, die vorzugsweise mit Klettverschlüssen ausgebildet sind, und wobei das zweite Paar Befestigungsbänder (124) die zweite Öffnung (190) flankiert.

10. Kopfbedeckung (100) nach einem der Ansprüche 1 bis 9, wobei das Textil (110) Faltlinien (170) aufweist, die geeignet sind, das Textil (110) an die Krümmung des Kopfes des Benutzers anzupassen.

## Revendications

1. Couvre-chef (100) destiné à être utilisé dans l'atténuation de céphalées de tension, de migraines et d'autres types de céphalées, ledit couvre-chef comprenant :
- une matière textile (110) ;
- un système de compartiments formés dans ou sur ladite matière textile (110), chaque compartiment contenant un matériau conçu pour être chauffé dans un four à micro-ondes à une température comprise entre 40 et 60 degrés Celsius ; et
- un moyen de fixation (120) conçu pour ajuster la matière textile à la courbure de la tête d'un utilisateur ;
dans lequel ledit système de compartiments est configuré pour cibler des muscles et/ou des nerfs spécifiques sur la tête de l'utilisateur avec de la chaleur, dans lequel ledit système de compartiments comprend une paire de compartiments d'un deuxième type (140) positionnés sur ledit couvre-chef (100) pour cibler les muscles occipitaux et le nerf occipital avec de la chaleur ; **caractérisé en ce que** ledit système de compartiments est configuré pour cibler des muscles et/ou des nerfs spécifiques sur la tête de l'utilisateur avec une chaleur comprise entre 40 à 60 degrés Celsius ; ledit système de compartiments comprenant également une paire de compartiments d'un premier type (130) positionnés sur ledit couvre-chef (100) pour cibler les muscles masséters et le nerf trijumeau ; et dans lequel la matière textile (110) est choisie parmi le coton, la laine, le lin, le chanvre et des mélanges de ceux-ci.

2. Couvre-chef (100) selon la revendication 1, dans lequel le système de compartiments comprend également :
- une paire de compartiments d'un troisième type (150) positionnés sur ledit couvre-chef (100) pour cibler le muscle temporal avec une chaleur comprise entre 40 à 60 degrés Celsius.

3. Couvre-chef (100) selon l'une quelconque des revendications 1 à 2, dans lequel le système de compartiments comprend également :
- un compartiment d'un quatrième type (160) positionné sur ledit couvre-chef pour cibler le muscle splénius de la tête avec une chaleur comprise entre 40 à 60 degrés Celsius.

4. Couvre-chef (100) selon l'une quelconque des revendications 1 à 2, dans lequel le système de compartiments comprend également :
- un ou plusieurs compartiments d'un quatrième type (160) positionnés sur ledit couvre-chef (100) pour cibler les muscles splénius de la tête, les muscles sternocléidomastoïdiens, la partie supérieure du muscle trapèze et/ou le nerf occipital avec une chaleur comprise entre 40 et 60 degrés Celsius.

5. Couvre-chef (100) selon l'une quelconque des revendications 1 à 4, comprenant également deux zones (170) définissant les zones correspondant aux oreilles de l'utilisateur, lesdites zones étant constituées uniquement de ladite matière textile, empêchant ainsi l'échauffement desdites oreilles.

6. Couvre-chef (100) selon l'une quelconque des revendications 1 à 5, configuré avec une première ouverture (180) définissant les zones correspondant à au moins une partie des régions frontale, médiane et le sommet du cuir chevelu de l'utilisateur.

7. Couvre-chef (100) selon la revendication 6, dans lequel le moyen de fixation (120) conçu pour ajuster la matière textile (110) à la courbure de la tête d'un utilisateur comprend une première paire de sangles de fixation (122), de préférence configurées avec des attaches auto-agrippantes, et dans lequel ladite première paire de sangles de fixation (122) borde ladite première ouverture (180).

8. Couvre-chef (100) selon l'une quelconque des revendications 1 à 6, configuré avec une seconde ouverture (190) définissant les zones correspondant à au moins une partie du front, des régions orbitale, nasale et orale de l'utilisateur.

9. Couvre-chef (100) selon la revendication 8, dans lequel le moyen de fixation (120) conçu pour ajuster la matière textile à la courbure de la tête d'un utilisateur comprend une seconde paire de sangles de fixation (124), de préférence configurées avec des attaches auto-agrippantes, et dans lequel ladite seconde paire de sangles de fixation (124) borde ladite seconde ouverture (190).

10. Couvre-chef (100) selon l'une quelconque des revendications 1 à 9, dans lequel la matière textile (110) comprend des lignes de pliage (170) conçues pour ajuster le tissu (110) à la courbure de la tête de l'utilisateur.
